# EUROPEAN PATENT APPLICATION

(11) **EP 4 821 430 A1**
(43) Date of publication of application: **14.10.2026**
(21) Application number: 26170240.1
(22) Date of filing: 01.04.2026
(51) Int. Cl.: A61B 5/024, A61B 5/1455

(54) **WEARABLE COMPUTING DEVICE HAVING A LIGHT BLOCKING SURFACE FOR A CALIBRATOR LIGHT DETECTOR**

(30) Priority: 11.04.2025 US 202519177197
(71) Applicant: Google LLC, Mountain View, CA 94043 (US)
(72) Inventor: Yao, Kaiyuan, Mountain View, 94043 (US); Vavadi, Hamed, Mountain View, 94043 (US); Winthrop Richards, Peter, Mountain View, 94043 (US); Pitale, Abhinuv Nitin, Mountain View, 94043 (US); Jung, Seobin, Mountain View, 94043 (US)
(74) Representative: Maikowski & Ninnemann Patentanwälte Partnerschaft mbB

(57) **Abstract**

A wearable computing device includes a housing with an upper housing portion and a lower housing portion. The lower housing portion includes a plurality of side walls defining an internal cavity, at least one light emitter positioned in the internal cavity, at least one light detector positioned in the internal cavity, at least one calibrator light detector positioned in the internal cavity, an outer cover arranged adjacent to the internal cavity, and a light blocking surface positioned between the at least one calibrator light detector and the outer cover so as to minimize transmittance of one or more interference signals to the at least one calibrator light detector.

## Description

### FIELD

The present disclosure relates generally to wearable computing devices, and more particularly, to a wearable computing device having a light blocking surface configured to minimize transmittance of one or more interference signals to a calibrator light detector.

### BACKGROUND

Many wearable computing devices have biometric sensors that rely on precise detection of a direct current (DC) level of optical emission and transmission intensity through a users' skin tissue. To maximize user value and provide longitudinal insight of health information, the biometric sensors must reliably measure small changes of DC signals over a prolonged period of time.

For example, skin autofluorescence (SAF), can be used by a wearable computing device to non-invasively detect and measure physiological data. Generally, SAF may be induced by illuminating the skin, such as with an excitation light. By illuminating the skin, SAF can be used to monitor the presence of advanced glycation end-products (AGEs), which are biomarkers present below the surface of skin associated with aging and cardiovascular health and have also been implicated in conditions such as diabetes, atherosclerosis, kidney disease, and Alzheimer's disease. In particular, SAF can be strongly correlated with health outcomes and can be used in conjunction with other physiological data (e.g., heart rate and oxygen saturation (SpO₂)) to provide information about an individual's health. However, determining the value of SAF requires monitoring small changes over time. For example, the expected monthly change of SAF and glucose level is on the order of 0.1% or smaller.

Typically, a light source or emitter (such as a light emitting diode (LED)) and a light detector (such as a photodiode (PD)) are utilized in wearable computing devices to monitor the small changes in SAF over time. However, the electrical-to-optical power conversion efficiency of an LED is known to degrade over time in a magnitude that is comparable to the expected signal change from physiological origins. Therefore, this LED aging behavior causes inconsistency within the DC optical emission and transmission signals, making it difficult to accurately detect small changes in the DC signals over a prolonged period of time and thus resulting in inaccurate biometric data.

To monitor the efficiency of the LED, a calibrator PD may be integrated into the wearable computing device. However, the calibrator PD may be impacted by external interference, such as signals from a user's skin and those reflected from the glass of the wearable device. These interference signals decrease the calibrator PD's ability to monitor the electrical-to-optical power conversion efficiency of the LED. As such, these external interference signals increase the difficulty of determining whether a particular LED has aged and prohibit effective continuous calibration while the wearable computing device is in operation.

As such, a need exists for a wearable computing device that addresses the aforementioned issues.

### SUMMARY OF THE INVENTION

Aspects and advantages of embodiments of the present disclosure will be set forth in part in the following description, or can be learned from the description, or can be learned through practice of the embodiments.

In an aspect, the present disclosure is directed to a wearable computing device having a housing which includes an upper housing portion and a lower housing portion. The lower housing portion of the wearable computing device includes a plurality of side walls defining an internal cavity. The wearable computing device also includes at least one light emitter positioned within the internal cavity, at least one light detector positioned within the internal cavity, and at least one calibrator light detector positioned within the internal cavity. The wearable computing device further includes an outer cover arranged adjacent to the internal cavity. In addition, the wearable computing device includes a light blocking surface positioned between the calibrator light detector(s) and the outer cover so as to minimize transmittance of one or more interference signals to the calibrator light detector(s).

In another aspect, the present disclosure is directed to an optical module of a computing device. The optical module includes a plurality of side walls defining an internal cavity. The optical module also includes at least one light emitter positioned within the first cavity portion and at least one calibrator light detector positioned within the first cavity portion adjacent to the light emitter(s). In addition, the optical module includes at least one light detector positioned within the second cavity portion. The optical module also includes an outer cover arranged adjacent to the internal cavity. Further, the optical module includes at least one light blocking surface positioned between the calibrator light detector(s) and the outer cover of the lower housing portion so as to minimize transmittance of one or more interference signals to the calibrator light detector(s).

These and other features, aspects, and advantages of various embodiments of the present disclosure will become better understood with reference to the following description and appended claims. The accompanying drawings, which are incorporated in and constitute a part of this specification, illustrate example embodiments of the present disclosure and, together with this description, serve to explain the related principles.

### BRIEF DESCRIPTION OF THE DRAWINGS

Detailed discussion of embodiments directed to one of ordinary skill in the art is set forth in the specification, which makes reference to the appended figures, in which:
FIG. 1A illustrates a perspective view of an embodiment of a wearable computing device in accordance with aspects of the present disclosure, particularly with the wearable computing device being worn on a wrist of a user.
FIG. 1B illustrates a side view of the wearable computing device of FIG. 1A in accordance with aspects of the present disclosure.
FIG. 2A illustrates a schematic view of an embodiment of a lower housing portion of a wearable computing device as viewed from inside the wearable computing device, particularly illustrating a plurality of light emitters, a plurality of light detectors, and a plurality of light blocking surfaces within an internal cavity of the lower housing portion in accordance with aspects of the present disclosure.
FIG. 2B illustrates a bottom view of the lower housing portion of the wearable computing device of FIG. 2A as viewed from a skin-side surface of the wearable computing device in accordance with aspects of the present disclosure.
FIG. 3 illustrates a side view of an embodiment of a lower housing portion of a wearable computing device having at least one light detector, at least one light emitter, at least one calibrator light detector, and a light blocking flange in accordance with aspects of the present disclosure.
FIG. 4A illustrates a side view of an embodiment of a lower housing portion of a wearable computing device having a switchable polarizing material in a transmissive state in accordance with aspects of the present disclosure.
FIG. 4B illustrates a side view of an embodiment of a lower housing portion of a wearable computing device having a switchable polarizing material in a light blocking state in accordance with aspects of the present disclosure.
FIGS. 5A and 5B illustrate side views of an embodiment of a lower housing portion of a wearable computing device having a switchable polarizing material and a light blocking flange in accordance with aspects of the present disclosure.
FIGS. 6A and 6B illustrate side views of various embodiments of an optical module for a computing device having a light blocking surface configured to reflect light emitted from a light emitter to a calibrator light detector in accordance with aspects of the present disclosure.

Repeated use of reference characters in the present specification and drawings is intended to represent the same or analogous features or elements of the present technology.

### DETAILED DESCRIPTION

Reference now will be made in detail to embodiments of the invention, one or more examples of which are illustrated in the drawings. Each example is provided by way of explanation of the invention, not limitation of the invention. In fact, it will be apparent to those skilled in the art that various modifications and variations can be made in the present invention without departing from the scope or spirit of the invention. For instance, features illustrated or described as part of one embodiment can be used with another embodiment to yield a still further embodiment. Thus, it is intended that the present invention covers such modifications and variations as come within the scope of the appended claims and their equivalents.

Health sensing applications rely on precise detection of the DC level of the optical emission and transmission intensity through a user's skin tissue to measure and monitor various biomarkers. Examples may include skin autofluorescence (SAF) sensing of advanced glycation end-products and multispectral sensing of glucose absorption. To provide longitudinal insight of various physiological data points, and therefore maximize value to the user, the sensors need to reliably detect and measure small changes in the DC signals over a prolonged time period.

For example, light emitting diodes (LEDs) may be used as the light source for wearable optical sensors. However, the power conversion efficiency of LEDs is known to degrade over time and becomes challenging to detect due to the microscale at which the signal changes over time. The effects of LED aging are known in wearable sensors that do not rely on DC optical signals (such as a PPG heart rate sensor). However, in DC-based optical sensors, such as SAF and multispectral sensing applications, the LED aging effects are not fully known.

As such, a calibrator light detector, such as a calibrator PD, may be integrated into the device to sense the DC level intensity of the LED in real time. However, the calibrator PD may be impacted by external interference signals, such as skin-reflected light or device-reflected light (e.g., from a glass or lens (if present) in the lower housing, from one or more walls defining an internal cavity of the lower housing, etc.). These interference signals decrease the reliability of the calibration signal and thus increase the difficulty of determining whether a particular LED has aged, which in turn prohibits effective continuous calibration while the device is in operation.

Accordingly, the present disclosure is directed to a wearable computing device that provides reliable and continuous on-wrist signal monitoring, such as for a DC-based optical sensor. In particular, the wearable computing device of the present disclosure includes a housing having an upper housing portion and a lower housing portion. The lower housing portion has a plurality of side walls defining an internal cavity. Further, the wearable computing device includes at least one light emitter (such as an LED) positioned in the internal cavity, at least one calibrator light detector (such as a calibrator PD) positioned in the internal cavity, an outer cover arranged adjacent to the internal cavity, and a light blocking surface positioned between the calibrator light detector(s) and the outer cover so as to minimize transmittance of one or more interference signals to the calibrator light detector(s). Thus, in an embodiment, the light blocking surface is configured to decrease the contributions from interference signals (such as signals from skin-reflected light and/or device-reflected light) to be insignificant as compared to the signal from the light emitted from a light emitter. As such, the signal detected by the calibrator light detector represents a more robust reflection of the light emitter efficiency and/or intensity.

Furthermore, in an embodiment, the plurality of side walls defining the internal cavity of the lower housing portion may define a first cavity portion and a second cavity portion that are separate from each other. Thus, in such embodiments, the light emitter(s) and the calibrator light detector(s) may be positioned in the first cavity portion, whereas the light detector(s) may be positioned in the second cavity portion.

In an embodiment, for example, the light blocking surface may be configured as a light blocking flange. Thus, in some embodiments, the light blocking flange may be formed integrally with one or more of the side walls defining the internal cavity of the lower housing portion. Alternatively, the light blocking flange may be a separate component attached to one or more of the side walls defining the internal cavity of the lower housing portion.

In additional embodiments, the light blocking surface may be constructed of a switchable polarizing material, wherein the switchable polarizing material can switch between a light transmittance state and a light blocking state. Accordingly, while in the light transmittance state, the switchable polarizing material is configured to allow light transmittance therethrough. In contrast, while in the light blocking state, the switchable polarizing material is configured to block light transmittance therethrough while in the blocking state. Thus, in an embodiment, the switchable polarizing material may form at least part of one or more of the plurality of side walls of the first cavity portion.

Moreover, in an embodiment, the light blocking surface may include both the light blocking flange and the switchable polarizing material as described herein. In such embodiments, the switchable polarizing material may be arranged to cover an opening in one or more of the plurality of side walls of the internal cavity. Furthermore, in such embodiments, the opening may be at least partially defined by the light blocking flange. Accordingly, the light blocking surface minimizes the interference signals from skin-reflected light and/or device-reflected light (e.g., from a glass or lens (if present) in the lower housing, from one or more walls defining the internal cavity of the lower housing, and/or from the light blocking surface itself).

With reference now to the Figures, example embodiments of the present disclosure will be discussed in further detail.

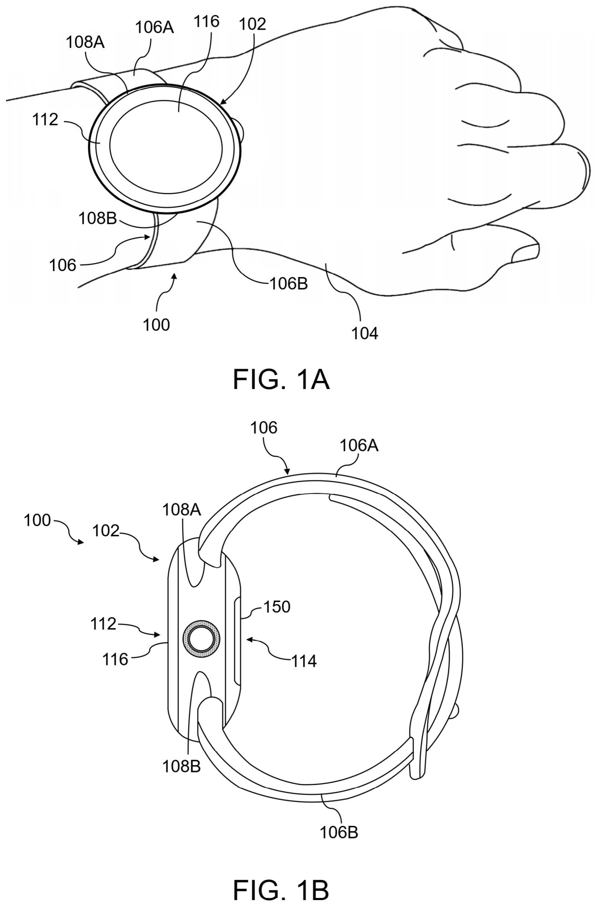

Referring now to the drawings, FIGS. 1A-1B illustrate various views of a wearable computing device 100 and components thereof according to the present disclosure. For instance, FIG. 1A illustrates a perspective view of the wearable computing device 100, particularly with the wearable computing device 100 being worn on a wrist of a user. FIG. 1B illustrates a side view of the wearable computing device 100 of FIG. 1A.

As generally shown in FIGS. 1A-1B, and as will be described below in greater detail, the wearable computing device 100 includes a housing 102 that contains electronics associated with the wearable computing device 100. In some instances, as shown in FIG. 1A, the wearable computing device 100 may be worn on a user's forearm 104 like a wristwatch. Thus, the wearable computing device 100 may include a wearable band, such as a wristband 106 having one or more parts, such as the two wristband straps 106A, 106B, for securing the wearable computing device 100 to the user's forearm 104. In such instance, the housing 102 may include one or more connection points (e.g., connection points 108A, 108B) at which the wristband 106 is couplable to the housing 102. For instance, the wristband 106 is shown coupled to the housing 102 at two locations (e.g., connection points 108A, 108B). However, it should be appreciated that the wearable computing device 100 may be worn at any other suitable location by a user, such as, for example, on an ankle, a torso, and/or the like.

In some embodiments, as shown in FIG. 1B, the housing 102 has an upper housing portion 112 and a lower housing portion 114 that together generally define an internal volume. Furthermore, the upper and lower housing portions 112, 114 are connectable to at least partially enclose the internal volume of the wearable computing device 100. Thus, the interior volume of the wearable computing device 100 is configured to at least partially receive one or more components. For instance, as shown in FIG. 1A, an electronic display screen 116 may be received within the internal volume and viewable through the upper housing portion 112. Further, in an embodiment, the upper housing portion 112 may include glass cover, polycarbonate cover, acrylic cover, or the electronic display screen 116. Moreover, in an embodiment, the electronic display screen 116 may cover an electronics package (not shown), which may also be housed within the internal volume of the housing 102. The electronic display screen 116 may be any suitable display type, such as a touch screen, organic light emitting diode (OLED), liquid crystal display (LCD), and/or the like.

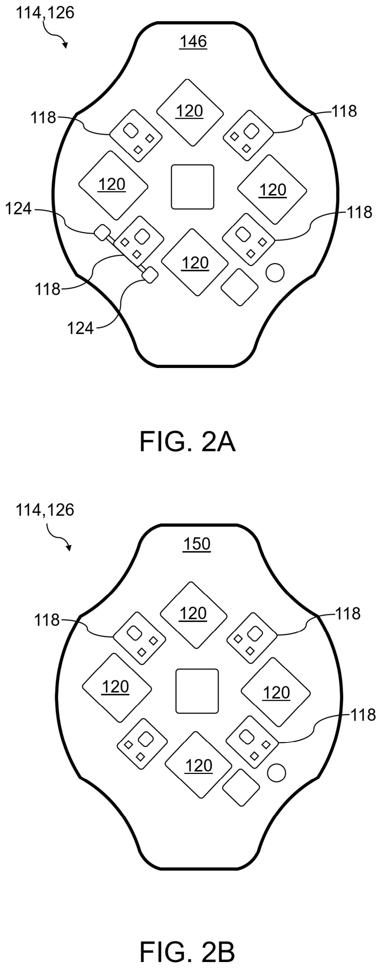

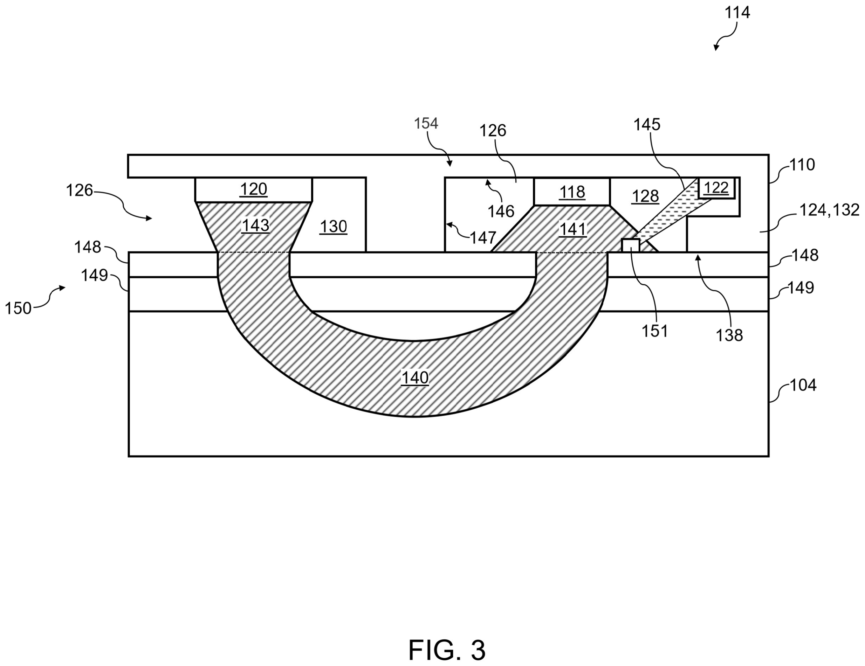

Moreover, the lower housing portion 114 may be configured to be closest to a user when worn and may generally include a plurality of side walls 110 defining an internal cavity 126 (FIGS. 2A-3). The lower housing portion 114 may also include an outer cover 150 configured to contact a dorsal wrist of a user when being worn by the user, as shown in FIG. 1A. Furthermore, in an embodiment, as shown in FIG. 3, the outer cover 150 includes one or more layers 148, 149 arranged exterior of the internal cavity 126. In such embodiments, for example, the layer(s) 148, 149 may be formed of any suitable material, such as glass, polymer, metal, etc., and/or combinations thereof. For example, the embodiment of FIG. 3 illustrates the outer cover 150 having an outermost layer 149 formed of glass and another intermediate layer 148 formed of plastic.

Referring now to FIGS. 2A, 2B, and 3, schematic views of an embodiment of the internal cavity 126 of the lower housing portion 114 of the wearable computing device 100 as viewed from inside (FIG. 2A), outside from a skin-side surface (FIG. 2B), and from the side (FIG. 3) so as to illustrate various components therein are illustrated in accordance with aspects of the present disclosure. In particular, as shown in FIG 2A, 2B, and 3, the lower housing portion 114 may further include an optical module 154 having a plurality of light emitters 118 (such as LEDs or UV LEDs), a plurality of light detectors 120 (such as PDs), at least one calibrator light detector 122 (such as a calibrator PD), and at least one light blocking surface 124 positioned between the calibrator light detector(s) 122 and the outer cover 150 so as to minimize transmittance of one or more interference signals to the calibrator light detector(s) 122. More specifically, as shown, such components are arranged generally in a circumferential arrangement. More particularly, as shown in FIGS. 2A and 2B, the plurality of light emitters 118 may be arranged in an alternating pattern with the plurality of light detectors 120.

In addition, as shown, the plurality of light emitters 118, the plurality of light detectors 120, and the calibrator light detector(s) 122 may be mounted or otherwise secured within the internal cavity 126 in any suitable manner. For example, as shown in FIGS. 2A and 3, the plurality of light emitters 118, the plurality of light detectors 120, and/or the calibrator light detector(s) 122 may be mounted to one of the side walls 110 (such as an upper side wall 146) defining the internal cavity 126. Moreover, as shown in FIG. 3, the internal cavity 126 may also define one or more cavity portions, such as a first cavity portion 128 and a second cavity portion 130 that are separated from each other (e.g., via one of the side walls 110, such as a divider wall 147). As such, in an embodiment, as shown particularly in FIG. 3, the light emitter(s) 118 and a corresponding calibrator light detector 122 may be positioned in the first cavity portion 128, whereas the light detector 120 may be positioned in the second cavity portion 130.

Such components may have any suitable arrangement, and it should be understood by those having ordinary skill in the art that the illustrated arrangement is provided as an example only and is not meant to be limiting. Furthermore, although only one of the light emitters 118 is illustrated as having one or more corresponding calibrator light detectors 122 and light blocking surface(s) 124, one having skill in the art would appreciate that any number of the light emitters 118 may have corresponding calibrator light detector(s) 122 and light blocking surface(s) 124 as described herein.

In an embodiment, the light detector(s) 120 described herein may be configured to detect ambient light (e.g., light from the surrounding environment not emitted from the wearable computing device 100) and/or at least a portion of the light 141 emitted by the light emitter(s) 118, that may be reflected from a user's skin. As such, in an embodiment, light 143 detected by the light detector(s) 120 may be used to determine an optical measurement of a biometric, such as a heart rate (HR) measurement, blood oxygen saturation (SpO₂) measurement, heart rate variability measurement, blood pressure, a SAF measurement, and/or other physiological metrics, and/or a distance of the lower housing portion 114 of the wearable computing device 100 from an object, such as the user's forearm 104 (FIG. 1A). In other embodiments, the light detector(s) 120 described herein may include a DC-based optical sensor, a temperature sensor (such as an ambient temperature sensor or a skin temperature sensor, a SAF sensor, a humidity sensor, a pressure sensor, one or more biometric sensors, or a combination thereof.

In an embodiment, the light emitter(s) 118 described herein may be used to emit light which may be transmitted through the outer cover 150 of the wearable computing device 100 and/or the skin of the user (such as the skin of the forearm 104 of a user). The light 141 emitted from the light emitter(s) 118 may be detected by the light detector(s) 120 or the calibrator light detector(s) 122 to provide an optical measurement of a biometric, as described above. More particularly, to calibrate the light emitter(s) 118, the light emitter(s) 118 may be driven at a constant current during a sensing application and a fraction of the light emitted (as represented by shaded region 145) may be detected by the calibrator light detector(s) 122. In embodiments, the light emitter(s) 118 described herein may include a light emitting diode (LED) or an ultraviolet (UV) LED.

Furthermore, as shown in the illustrated embodiment in FIGS. 2A, 2B, and 3, the light blocking surface 124 is designed as a light blocking flange 132 positioned between the calibrator light detector 122 and the outer cover 150 to create a "dark house" in the mechanical structure of the lower housing portion 114, thereby hiding the calibrator light detector 122 inside the dark house. Additionally, as shown in FIG. 2A, an inside view of the lower housing portion 114, i.e., with the layers 148, 149 of the outer cover 150 (FIGS. 2B and 3) removed to better illustrate the internal components described herein is illustrated; whereas FIG. 2B illustrates an outside view of the lower housing portion 114, i.e., with the layers 148, 149 of the outer cover 150 in place to better illustrate how the light blocking surface 124 described herein functions.

More specifically, as shown in FIG. 3, light emitted by the light emitter 118 is represented by shaded area 141, light detected by the light detector 120 is represented by shaded area 143, signals passing through the glass and/or skin are represented by shaded area 140, and light detected by the calibrator light detector 122 is represented by shaded area 145. During a sensing application, in an embodiment, the light emitter 118 is driven at constant current and a fraction of the emitted light 141 is reflected back to the calibrator light detector 122, e.g., via a reflective mechanism 151 (again, represented by shaded region 145). It will be appreciated that the exemplary reflective mechanism 151 of FIG.3 is provided by way of example only, and that in other exemplary embodiments, any other suitable reflective mechanism 151 may be provided. For example, although the exemplary reflective mechanism 151 includes a reflective prism, in other exemplary embodiments of the present disclosure the reflective mechanism 151 may alternatively include a surface with a reflective coating or solution, a mirror, or an optical component. Additionally or alternatively, although the exemplary reflective mechanism 151 is depicted as being present in the internal cavity 126 of the lower housing portion 114, it will be appreciated that the reflective mechanism 151 may not be present. For example, when the reflective mechanism 151 is not present in the internal cavity 126 of the lower housing portion 114, the light blocking surface 124, such as a light blocking flange 132, may be utilized to reflect light emitted 141 from the light emitter 118 to the calibrator light detector 122. Alternatively or additionally, at least one layer of the outer cover 150 of the lower housing portion 114 may be configured to reflect light emitted 141 from the light emitter 118 to the calibrator light detector 122.

The photocurrent can be recorded by the transimpedance amplifier circuit in an analog front end to provide the calibration signal. By careful dark house design, the contributions from the interference signal(s) 140 (e.g., skin-reflected light and/or device-reflected light) are small enough compared to the internal calibration pathway, so that the calibration signal is a robust reflection of internal light emitter 118 efficiency. More specifically, as shown, the light blocking surface 124 (e.g., the light blocking flange 132) is configured to minimize transmittance of the interference signal(s) 140 to the calibrator light detector(s) 122 so as to improve accuracy of calibrating the corresponding light emitter 118 during operation.

In an embodiment, as shown in FIG. 3, the light blocking flange 132 may be formed integrally with one or more of the plurality of side walls 110 defining the internal cavity 126. In another embodiment, the light blocking flange 132 may be a separate component attached to one or more of the plurality of side walls 110 using any suitable attachment means, such as via fasteners, adhesives, etc. Thus, the light blocking flange 132 may define at least a portion of an exterior surface 138 of the internal cavity 126.

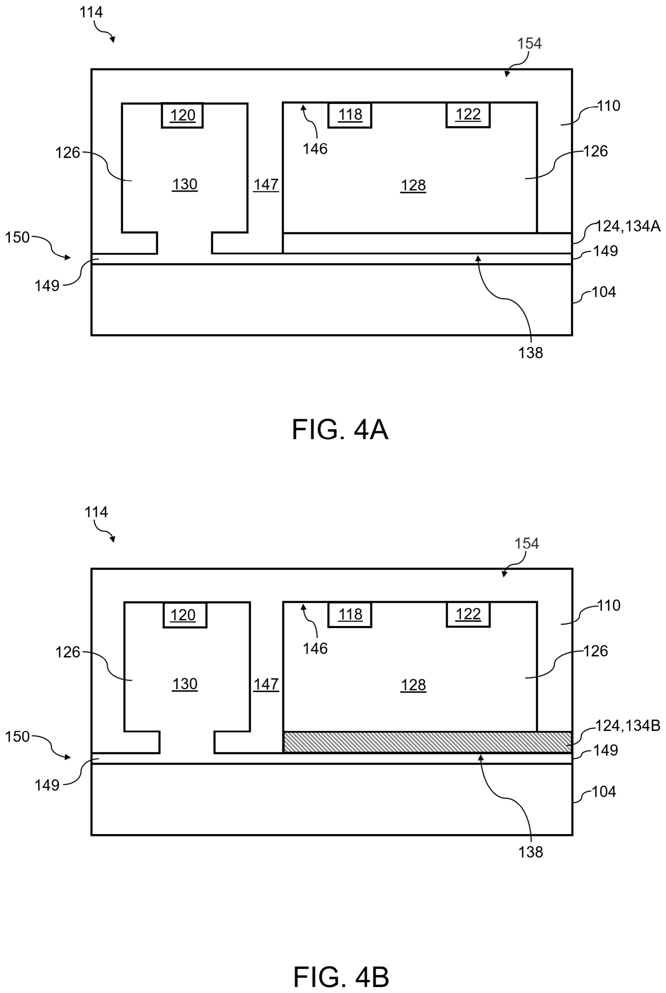

Referring now to FIGS. 4A-4B, side views of another embodiment of the internal cavity 126 of the wearable computing device 100 having the light blocking surface 124 configured as a switchable polarizing material 134 are illustrated in accordance with aspects of the present disclosure. More specifically, FIG. 4A illustrates the switchable polarizing material 134 in a light transmittance state 134A, whereas FIG. 4B illustrates the switchable polarizing material 134 in a light blocking state 134B. Further, as shown, the switchable polarizing material 134 is generally positioned between the first cavity portion 128 and the outer cover 150 and thus forms at least part of the exterior surface 138 of the internal cavity 126. In such embodiments, the switchable polarizing material 134 can switch states using electrically switchable polarizers. In particular, in an embodiment, the wearable computing device 100 is configured to allow a voltage to control window transmission within a microseconds time scale to enable switching between the light transmittance state 134A (FIG. 4A) and the light blocking state 134B (FIG. 4B) for dark calibration without external interference signals. Furthermore, in an embodiment, the switchable polarizing material 134 can remain OFF (e.g., in the light transmittance state 134A) during normal operation of the wearable computing device 100 but can be turned ON (e.g., in the light blocking state 134B) during calibration operation of the wearable computing device 100. Such operation is configured to ensure that the light emitter 118 and the corresponding calibrator light detector(s) 122 are hidden from interference signals during a calibration operation. In particular embodiments, the switchable polarizing material 134 is configured to provide near 100% transmissivity when OFF, near 100% absorption when ON, and can switch between ON/OFF states in order of microseconds.

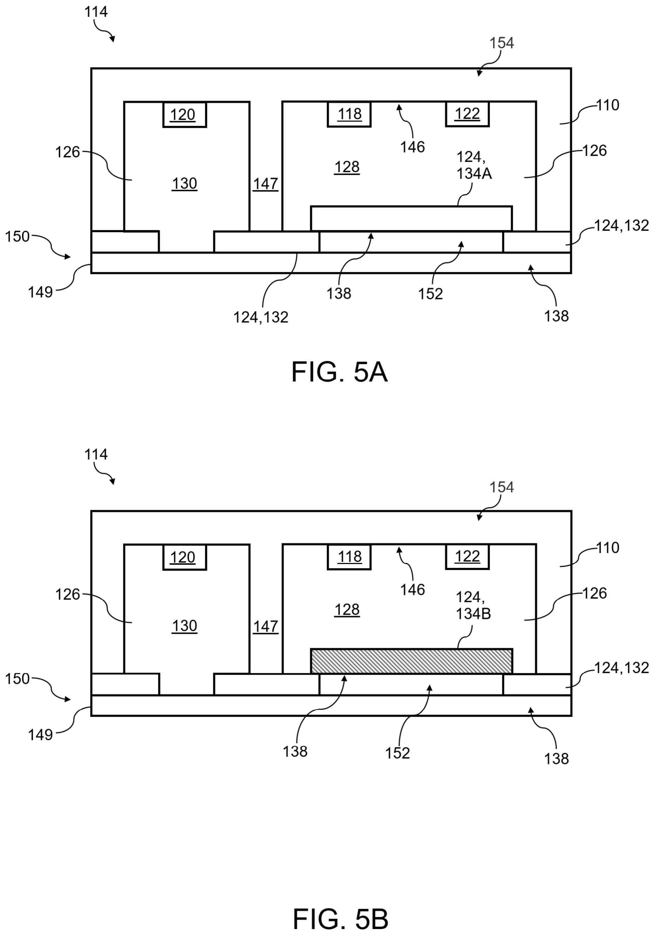

Referring now to FIGS. 5A-5B, side views of yet another embodiment of the lower housing portion 114 of the wearable computing device 100 having the switchable polarizing material 134 and the light blocking flange 132 is illustrated in accordance with aspects of the present disclosure. More particularly, as shown, the light blocking flange 132 is positioned between the calibrator light detector 122 and the outer cover 150 of the lower housing portion 114 to create a "dark house" in the mechanical structure of lower housing portion 114, thereby hiding the calibrator light detector 122 inside the dark house.

Furthermore, as depicted in FIGS. 5A and 5B, the switchable polarizing material 134 may be positioned adjacent to the light blocking flange 132, with at least a portion of the switchable polarizing material 134 resting atop the light blocking flange 132. More specifically, as shown, the switchable polarizing material 134 may be arranged within the first cavity portion 128 to cover an opening 152 in the exterior surface 138 of the internal cavity 126. Furthermore, in such embodiments, as shown, the light blocking flange 132 may be formed integrally with one or more of the plurality of side walls 110 defining the internal cavity 126. In addition or alternatively, as mentioned, the light blocking flange 132 may be a separate component attached or otherwise secured to one or more of the plurality of side walls of the lower housing portion 114. Thus, as shown, the light blocking flange 132 and/or the switchable polarizing material 134 may form at least part of the exterior surface 138 of the internal cavity 126.

In such embodiments, as described herein, the switchable polarizing material 134 is configured to switch between the light transmittance state 134A (FIG. 5A) and the light blocking state 134B (FIG. 5B), e.g., using electrically switchable polarizers. In particular, in an embodiment, the wearable computing device 100 is configured to use voltage to control the window transmission within microseconds time scale to enable switching between the light transmittance state 134A (FIG. 5A) and the light blocking state 134B (FIG. 5B) for dark calibration without external interference signals.

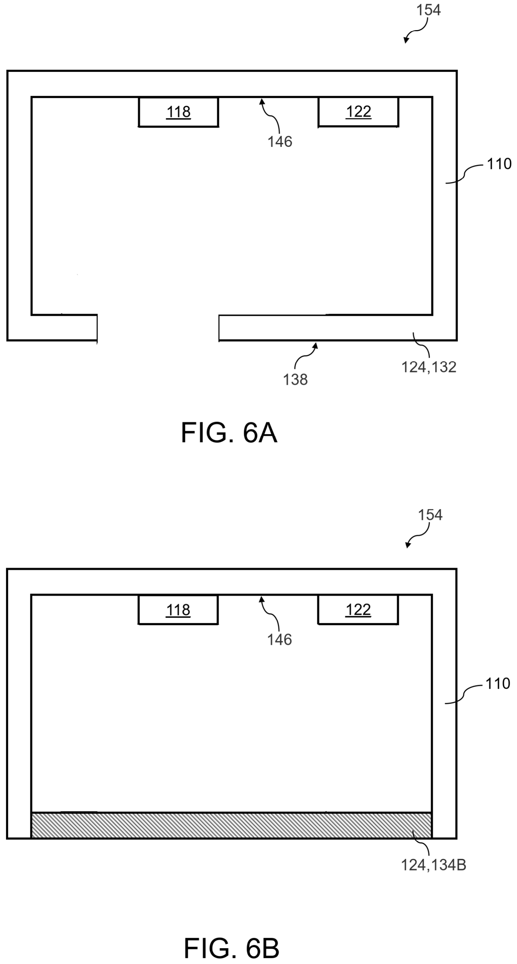

Referring now to FIGS. 6A-6B, side views of various embodiments of the optical module 154 for a computing device is illustrated. As described herein, the optical module 154 may be part of or integrated with the lower housing portion 114 of the wearable computing device 100. Further, as shown in FIG. 6A, the optical module 154 includes the light blocking surface 124 configured as the light blocking flange 132, whereas FIG. 6B illustrates the light blocking surface 124 configured as the switchable polarizing material 134. Thus, in such embodiments, FIGS. 6A-6B illustrate the optical module 154 configured as an LED component (e.g., such as an LED component packaging) for use or implementation in a computing device, such as the wearable computing device 100. Additionally, the optical module 154 may include at least one light emitter 118 and at least one calibrator light detector 122. The light emitter(s) 118 and calibrator light detector(s) 122 may be configured as described above in FIGS. 2A-5B. Furthermore, the optical module 154 includes a light blocking surface 124 configured to minimize transmittance of one or more interference signals to the calibrator light detector(s) 122. In an embodiment, as described herein, the light blocking surface 124 may be integrated into the computing device described herein. In such embodiments, the light blocking surface 124 may be positioned between the calibrator light detector(s) 122 and a side wall 110 of the lower housing portion 114 so as to minimize transmittance of one or more interference signals to the calibrator light detector(s) 122. Thus, in such embodiments, the light blocking surface 124 may be integrally formed with one or more of the plurality of side walls 110. In another embodiment, the light blocking surface 124 may be separate and therefore attached to one or more of the plurality of side walls 110 using any suitable attachment means, as described above.

Alternatively, or additionally, the light blocking surface 124 of the optical module 154 may be configured as a switchable polarizing material 134, as shown in FIG. 6B, and further described herein.

While the present disclosure has been described in detail with respect to various specific example embodiments thereof, each example is provided by way of explanation, not limitation of the disclosure. Those skilled in the art, upon attaining an understanding of the foregoing, can readily produce alterations to, variations of, and equivalents to such embodiments. Accordingly, the subject disclosure does not preclude inclusion of such modifications, variations and/or additions to the present disclosure as would be readily apparent to one of ordinary skill in the art. For instance, features illustrated or described as part of one embodiment can be used with another embodiment to yield a still further embodiment. Thus, it is intended that the present disclosure covers such alterations, variations, and equivalents.

## Claims

1. A wearable computing device, comprising:
a housing comprising an upper housing portion and a lower housing portion, the lower housing portion comprising:
a plurality of side walls defining an internal cavity;
at least one light emitter positioned in the internal cavity;
at least one light detector positioned in the internal cavity;
at least one calibrator light detector positioned in the internal cavity;
an outer cover arranged adjacent to the internal cavity; and
a light blocking surface positioned between the at least one calibrator light detector and the outer cover so as to minimize transmittance of one or more interference signals to the at least one calibrator light detector.

2. The wearable computing device of claim 1, wherein the light blocking surface comprises a light blocking flange.

3. The wearable computing device of claim 2, wherein the light blocking flange is formed integrally with one or more of the plurality of side walls of the lower housing portion.

4. The wearable computing device of claim 2, wherein the light blocking flange is a separate component attached to one or more of the plurality of side walls of the lower housing portion.

5. The wearable computing device of any one of the preceding claims, wherein the light blocking surface is constructed of a switchable polarizing material, wherein the switchable polarizing material switches between a light transmittance state and a light blocking state.

6. The wearable computing device of claim 5, wherein the switchable polarizing material forms at least part of one or more of the plurality of side walls of the internal cavity.

7. The wearable computing device of claim 6, wherein the light blocking surface comprises a light blocking flange and a switchable polarizing material, the switchable polarizing material arranged to cover an opening in one or more of the plurality of side walls of the internal cavity.

8. The wearable computing device of any one of the preceding claims, wherein the plurality of side walls defining the internal cavity define a first cavity portion and a second cavity portion, the first cavity portion and the second cavity portion separate from each other, the at least one light emitter and the at least one calibrator light detector being positioned in the first cavity portion, the at least one light detector being positioned in the second cavity portion.

9. The wearable computing device of any one of the preceding claims, wherein the one or more interference signals comprise at least one of skin-reflected light, device-reflected light, or a combination thereof.

10. The wearable computing device of any one of the preceding claims, wherein the at least one light emitter comprises a light emitting diode. LED, an ultraviolet, UV, LED, or a combination thereof.

11. The wearable computing device of any one of the preceding claims, wherein at least one of the at least one light detector or the at least one calibrator light detector comprises a photodiode.

12. An optical module of a computing device, the optical module comprising:
a plurality of side walls defining an internal cavity;
at least one light emitter positioned within the internal cavity;
at least one calibrator light detector positioned within the internal cavity adjacent to the at least one light emitter; and
at least one light blocking surface positioned between the at least one calibrator light detector and at least one of the plurality of side walls defining the internal cavity so as to minimize transmittance of one or more interference signals to the at least one calibrator light detector.

13. The optical module of claim 12, wherein the light blocking surface comprises a light blocking flange.

14. The optical module of claim 12 or 13, wherein the light blocking surface is constructed of a switchable polarizing material, wherein the switchable polarizing material switches between a light transmittance state and a light blocking state.

15. The optical module of any one of claims 12 to 14, wherein the light blocking surface comprises a light blocking flange and a switchable polarizing material, the switchable polarizing material arranged to cover an opening in one or more of the plurality of side walls of the internal cavity.
